**Europäisches Patentamt**

**European Patent Office**

**Office européen des brevets**

(19)

(11) Numéro de publication : **0 330 532 B1**

(12)

# FASCICULE DE BREVET EUROPEEN

(45) Date de publication du fascicule du brevet :
**16.12.92 Bulletin 92/51**

(51) Int. Cl.⁵ : **A61K 9/16,** A61K 9/48,
A61K 47/00, A61K 31/215

(21) Numéro de dépôt : **89400247.6**

(22) Date de dépôt : **30.01.89**

(54) **Nouvelle forme galénique du fénofibrate.**

(30) Priorité : **26.02.88 FR 8802359**

(43) Date de publication de la demande :
**30.08.89 Bulletin 89/35**

(45) Mention de la délivrance du brevet :
**16.12.92 Bulletin 92/51**

(84) Etats contractants désignés :
**AT BE CH DE ES FR GB GR IT LI LU NL SE**

(56) Documents cités :
**EP-A- 0 179 583**
**EP-A- 0 239 541**
**EP-A- 0 256 933**
**WO-A-82/01649**

(73) Titulaire : **FOURNIER INDUSTRIE ET SANTE**
**38, avenue Hoche**
**F-75008 Paris (FR)**

(72) Inventeur : **Curtet, Bernard**
**57, rue du Carré**
**F-21160 Marsannay la Cote (FR)**
Inventeur : **Teillaud, Eric**
**1, allée Roger Bernard**
**F-21240 Talant (FR)**
Inventeur : **Reginault, Philippe**
**13, rue de Provence**
**F-21121 Fontaine les Dijon (FR)**

(74) Mandataire : **Clisci, Serge et al**
**S.A. FEDIT-LORIOT & AUTRES 38, Avenue**
**Hoche**
**F-75008 Paris (FR)**

**EP 0 330 532 B1**

Jouve, 18, rue Saint-Denis, 75001 PARIS

## Description

La présente invention concerne une nouvelle forme galénique du fénofibrate. Elle vise plus précisément une composition thérapeutique renfermant le fénofibrate et assurant une biodisponibilité améliorée, et un procédé pour la préparation de cette composition.

Le fénofibrate (dénomination commune internationale), préconisé dans le traitement des hyperlipidémies et des hypercholestérolémies répond à la nomenclature de 2-(4-(4-chlorobenzoyl)-phénoxy)-2-méthyl-propionate d'isopropyle. La posologie usuelle chez l'homme adulte est de trois gélules par jour, chacune dosée à 100 mg de fénofibrate.

Pour le confort du patient, il est avantageux de rechercher une forme galénique ne nécessitant qu'une seule prise journalière et qui permette un effet physiologique identique à celui obtenu lors de prises multiples. Il a donc été proposé une gélule dosée à 300 mg de fénofibrate, la posologie alors préconisée étant d'une seule prise par jour.

On peut cependant chercher à améliorer encore la forme galénique. On sait en effet que la biodisponibilité du fénofibrate n'est pas égale à 100 %. Il est donc souhaitable de mettre au point une forme galénique présentant une biodisponibilité améliorée du fénofibrate et permettant une administration unique quotidienne.

On sait que la micronisation d'un principe actif peut permettre d'améliorer in vivo la dissolution et par suite la biodisponibilité dudit principe actif. On sait encore que l'adjonction d'un excipient tensio-actif à une formulation d'un principe actif peut permettre d'améliorer l'absorption, et par conséquence la biodisponibilité dudit principe actif.

On vient à présent de découvrir que la co-micronisation du fénofibrate et d'un tensio-actif solide, (c'est-à-dire la micronisation d'un mélange intime de fénofibrate et d'un tensio-actif solide) permet d'améliorer la biodisponibilité du fénofibrate de façon significativement plus importante que l'amélioration que l'on obtiendrait soit par addition d'un agent tensio-actif, soit en micronisant uniquement le fénofibrate, soit encore en mélangeant intimement le fénofibrate et le tensio-actif micronisés séparément.

La présente invention propose donc une nouvelle composition thérapeutique, utile notamment dans le traitement par voie orale des hyperlipidémies et des hypercholestérolémies, caractérisée en ce qu'elle comporte du fénofibrate et un agent tensio-actif solide co-micronisés.

La quantité de fénofibrate préconisée est d'environ 200 mg par unité thérapeutique.

L'agent tensio-actif sera choisi parmi les tensio-actifs solides de façon à pouvoir le co-microniser avec le fénofibrate. On préférera un sulfate alcalin de l'alcool laurylique, comme par exemple le lauryl-sulfate de sodium (autre dénomination : dodécyl-sulfate de sodium). La quantité de laurylsulfate de sodium préconisée sera comprise entre 0,5 % et 7 % en poids par rapport au poids total de la formulation. De façon avantageuse, le rapport pondéral agent tensio-actif/fénofibrate sera compris entre environ 0,75/100 et 10,5/100.

La co-micronisation du fénofibrate et du tensio-actif solide sera réalisée avantageusement dans un broyeur à jet d'air accéléré jusqu'à obtenir une poudre telle que la taille moyenne des particules soit inférieure à 15 μm, de préférence inférieure à 10 μm, et encore mieux inférieure à 5 μm.

Pour obtenir une poudre formulable en gélules, on pourra ajouter au co-micronisat de fénofibrate et du tensio-actif solide des excipients classiques de remplissage, de dispersion et d'écoulement, comme par exemple le lactose, l'amidon, la polyvinylpyrrolidone et le stéarate de magnésium.

Selon l'invention, on préconise un procédé de préparation d'une composition thérapeutique contenant du fénofibrate et un agent tensio-actif solide caractérisé en ce que :

(i) on mélange intimement puis co-micronise le fénofibrate et l'agent tensio-actif solide,

(ii) on additionne au mélange obtenu du lactose et de l'amidon,

(iii) on granule l'ensemble en présence d'eau,

(iv) on sèche jusqu'à obtention d'un granulé contenant au plus 1 % d'eau,

(v) on calibre le granulé,

(vi) on additionne au granulé calibré de la polyvinylpyrrolidone et du stéarate de magnésium,

L'invention sera mieux comprise à la lecture des exemples de préparation qui suivent ainsi qu'à la lecture des résultats obtenus lors de tests comparatifs qui montrent la non-évidence de l'invention.

## PREPARATION I

Pour 100 000 gélules, chacune pesant 350 mg et renfermant 200 mg de fénofibrate, les quantités de produits utilisés sont les suivantes :

| | |
|---|---|
| fénofibrate | :20,0 kg |
| laurylsulfate de sodium | :0,7 kg |
| α-lactose monohydraté | :10,1 kg |

| amidon prégélatinisé | :3,0 kg |
| polyvinylpyrrolidone réticulée | :0,7 kg |
| stéarate de magnésium | :0,5 kg |

On co-micronise le mélange fénofibrate-laurylsulfate de sodium dans un microniseur à jet d'air de façon à obtenir une poudre dont la médiane de micronisation est de 3 $\mu$m. On additionne ensuite à cette poudre le lactose et l'amidon et on granule l'ensemble en présence de 8,9 % d'eau distillée par rapport au poids total du mélange. Le granulé ainsi obtenu est séché une journée à 50°C, puis on calibre le granulé pour ne retenir que les particules de tailles inférieures ou égales à 1000 $\mu$m. On additionne alors la polyvinylpyrrolidone et le stéarate de magnésium et on mélange l'ensemble jusqu'à homogénéité. La poudre obtenue sert à remplir des gélules de taille N° 1 sur une machine automatique avec un tassement réglé à un maximum de 150 N.

## PREPARATION II

On procède comme indiqué à la préparation en utilisant un mélange fénofibrate-laurylsulfate de sodium dont la médiane de micronisation est 6-7 $\mu$m.

## PREPARATION III

Pour 100 000 gélules de taille 1, chacune pesant 297 mg et renfermant 200 mg de principe actif, les quantités de produits utilisés sont les suivantes :

| fénofibrate | :20,0 kg |
| laurylsulfate de sodium | :0,3 kg |
| $\alpha$-lactose monohydraté | : 6,8 kg |
| amidon prégélatinisé | :1,5 kg |
| polyvinylpyrrolidone réticulée | : 0,6 kg |
| stéarate de magnésium | : 0,5 kg |

On procède de façon analogue à la préparation I, la co-micronisation du mélange fénofibrate-laurylsulfate de sodium étant telle que la médiane de micronisation est 6-7 $\mu$m et la granulation étant conduite en présence de 10 % d'eau distillée par rapport au poids du mélange fénofibrate-laurylsulfate de sodium-lactose-amidon.

## PREPARATION IV

De façon analogue à la procédure décrite à la préparation I en utilisant un mélange co-micronisé de fénofibrate et de laurylsulfate de sodium dont la médiane de micronisation est 6-7 $\mu$m, on a préparé les formulations regroupées dans le tableau I suivant :

## TABLEAU I

### COMPOSITION (en mg) PAR GELULE

| INGREDIENT | FORMULATION | | | | | |
|---|---|---|---|---|---|---|
| | A | B | C | D | E | F |
| Fénofibrate | 200 | 200 | 200 | 200 | 200 | 200 |
| Laurylsulfate de Na | 0 | 3 | 7 | 12 | 17,5 | 26,5 |
| Lactose | 108 | 105 | 101 | 95 | 90,5 | 83,5 |
| Amidon | 30 | 30 | 30 | 30 | 30 | 30 |
| Polyvinylpyrrolidone | 7 | 7 | 7 | 7 | 7 | 7 |
| Stéarate de Mg | 5 | 5 | 5 | 5 | 5 | 5 |
| Pourcentage en laurylsulfate de Na | 0 | 0,86 | 2 | 3,4 | 5 | 7,53 |

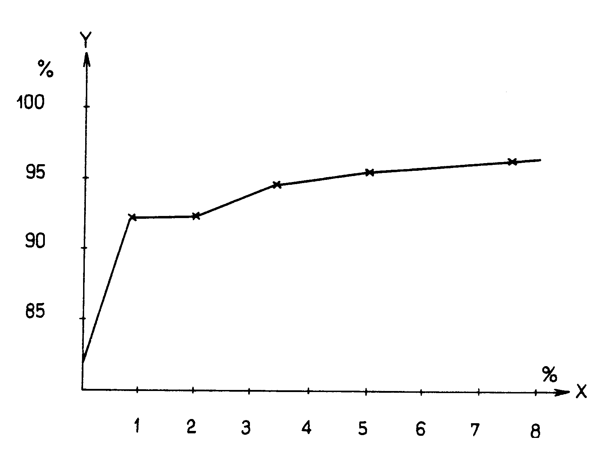

A partir de ces formulations, on a tracé la courbe de dissolution représentée à la figure 1 où la quantité en pourcentage de fénofibrate (Y) dissous est donnée en fonction du pourcentage de laurylsulfate de sodium (X) contenu dans la formulation. Les cinétiques de dissolution sont déterminées, conformément à la Pharmacopée Européenne, avec un appareil à palettes tournantes, l'éluant étant constitué d'eau et de laurylsulfate de sodium 0,1 M. Le fénofibrate est dosé par spectrophotométrie UV à 282 nm. La courbe de la figure 1 est donnée par les valeurs obtenues au temps 20 minutes.

Il ressort de ces résultats qu'à la concentration de 0 % de laurylsulfate de sodium, 82 % de fénofibrate sont dissous, à la concentration de 0,5 %, 87 % de fénofibrate sont dissous, à la concentration de 1 %, 92 % de fénofibrate sont dissous, et on obtient une dissolution maximale de 95 à 96 % de fénofibrate à partir de 4 % de laurylsulfate de sodium.

On a également tracé les courbes de dissolution, en cellule à flux continu avec un débit de 20 ml/min de laurylsulfate de sodium 0,1M, pour des formulations contenant du fénofibrate et du laurylsulfate de sodium (LSNa) co-micronisés comparativement au fénofibrate micronisé et à des formulations obtenues en mélangeant intimement le fénofibrate et le laurylsulfate micronisés séparément. La comparaison est réalisée sur T 50 %, c'est-à-dire le temps nécessaire pour dissoudre 50 % de fénofibrate. Les résultats obtenus sont consignés dans le tableau II suivant :

## TABLEAU II

## VALEUR DES TEMPS T 50 % (en minutes)

| INGREDIENTS | A | B | C |
|---|---|---|---|
| Fénofibrate pur micronisé | 37,165 | 37,165 | 0 |
| Fénofibrate + 1 % LSNa | 18,01 | 8,62 | −52,14 |
| Fénofibrate + 3 % LSNa | 23,75 | 12,68 | −46,61 |
| Fénofibrate + 5 % LSNa | 20,35 | 11,425 | −43,86 |
| Fénofibrate + 7 % LSNa | 14,5 | 10,76 | −25,79 |

**Notes**

A : mélange de micronisats

B : co-micronisation du mélange des ingrédients

C : variation $\dfrac{B - A}{A} \times 100$ (en %)

Il ressort de ces résultats que le T 50 % du fénofibrate est très significativement diminué (donc la vitesse de dissolution du fénofibrate est très significativement augmentée) quand l'on co-micronise le fénofibrate et le laurylsulfate de sodium par rapport au mélange de fénofibrate et de laurylsulfate de sodium micronisés séparément et par rapport au fénofibrate seul.

La vitesse de dissolution du fénofibrate est corrélée à la biodisponibilité du fénofibrate qui augmente quand la vitesse de dissolution augmente. Les résultats précédents montrent qu'il n'était pas à la portée de l'homme de l'art de réaliser une composition thérapeutique caractérisée par la co-micronisation du fénofibrate et d'un tensio-actif solide.

Ces résultats ont été confirmés en clinique. On a administré à des lots de sujets sains du fénofibrate (a) sous la forme d'une prise unique (1 gélule) de 300 mg de fénofibrate non micronisé (commercialisé sous le nom de marque "LIPANTHYL 300") et (b) sous la forme d'une prise unique de 200 mg de fénofibrate co-micronisé obtenu selon la préparation III sus-décrite. On prélève le sang des sujets à intervalles réguliers et dose un des métabolites actifs, l'acide 2-[4-(4-chlorobenzoyl)-phénoxy]-2-méthylpropionique. On trace la courbe de concentration de ce métabolite en fonction du temps et calcule l'aire sous la courbe [AUC(o-∞)] exprimée en mg/l.h.

Les résultats obtenus figurent dans le tableau III ci-après :

## TABLEAU III

| PARAMETRE DE BIODISPONIBILITE | FENOFIBRATE 200 mg (1) | FENOFIBRATE 300 mg (2) |
|---|---|---|
| AUC(o-∞)(mg/l.h) | 174,15 ± 48,67 | 168,85 ± 57,68 |
| C max (mg/l) | 10,86 ± 2,13 | 10,39 ± 2,89 |
| t max (h) | 5,97 ± 2,50 | 5,52 ± 1,70 |
| t 1/2 (h) | 15,13 ± 4,27 | 17,79 ± 8,77 |

<u>Notes</u>

(1) fénofibrate co-micronisé (200 mg)

(2) fénofibrate non micronisé (300 mg)

Les résultats du tableau III montrent qu'il n'y a pas de différence statistiquement significative entre la biodisponibilité in vivo de 200 mg de fénofibrate co-micronisé suivant l'invention et de 300 mg de fénofibrate non micronisé (qui est à l'heure actuelle la forme galénique préférée pour une administration quotidienne unique). En d'autres termes, le fénofibrate co-micronisé et dosé à 200 mg est bioéquivalent au fénofibrate non micronisé dosé à 300 mg.

Selon un autre aspect de l'invention, on préconise un procédé pour améliorer la biodisponibilité du fénofibrate in vivo, ledit procédé étant caractérisé en ce qu'il comprend la co-micronisation du fénofibrate et d'un agent tensio-actif solide, ladite co-micronisation étant réalisée par micronisation d'un mélange fénofibrate-/agent tensio-actif solide jusqu'à obtention d'une poudre de granulométrie inférieure à 15 µm et mieux inférieure ou égale à 5 µm.

## Revendications

**Revendications pour les Etats contractants suivants : AT, BE, CH, DE, FR, GB, IT, LI, LU, NL, SE**

1. Composition thérapeutique, utile notamment dans le traitement par voie orale des hyperlipidémies et des hypercholestérolémies caractérisée en ce qu'elle comporte du fénofibrate et un agent tensio-actif solide co-micronisés.

2. Composition thérapeutique selon la revendication 1, caractérisée en ce que le rapport pondéral agent tensio-actif/fénofibrate est compris entre 0,75/100 et 10,5/100.

3. Composition thérapeutique selon la revendication 1 caractérisée en ce que la quantité de fénofibrate est égale à 200 mg par unité thérapeutique.

4. Composition thérapeutique selon la revendication 1 caractérisée en ce que l'agent tensio-actif solide est le laurylsulfate de sodium.

5. Composition thérapeutique selon la revendication 4 caractérisée en ce que la quantité de lauylsulfate de sodium est comprise entre 0,5 et 7 % en poids par rapport au poids total de la formulation.

6. Composition thérapeutique selon la revendication 1 caractérisée en ce que la taille moyenne des particules de fénofibrate et de laurylsulfate de sodium co-micronisés est inférieure à 15 $\mu$m, de préférence inférieure ou égale à 10 $\mu$m, et mieux inférieure ou égale à 5 $\mu$m.

7. Composition thérapeutique selon l'une quelconque des revendications 1 à 6 caractérisée en ce qu'elle comporte en outre des excipients tels que des agents de dispersion, de remplissage et d'écoulement.

8. Procédé de fabrication d'une composition thérapeutique selon la revendication 1 caractérisé en ce que :
(i) on mélange intimement puis co-micronise le fénofibrate et un agent tensio-actif solide,
(ii) on additionne au mélange obtenu du lactose et de l'amidon,
(iii) on granule l'ensemble en présence d'eau,
(iv) on sèche jusqu'à obtention d'un granulé contenant au plus 1 % d'eau,
(v) on calibre le granulé, et
(vi) on additionne de la polyvinylpyrrolidone et du stéarate de magnésium.

9. Procédé selon la revendication 8 caractérisé en outre par le fait que la taille moyenne des particules de fénofibrate et de laurylsulfate de sodium co-micronisés est inférieure à 15 $\mu$m.

10. Procédé pour améliorer la biodisponibilité du fénofibrate in vivo, ledit procédé étant caractérisé en ce qu'il comprend la co-micronisation du fénofibrate et d'un agent tensio-actif solide, ladite co-micronisation étant réalisée par micronisation d'un mélange fénofibrate/agent tensio-actif solide jusqu'à obtention d'une poudre de granulométrie inférieure à 15 $\mu$m et mieux inférieure ou égale à 5 $\mu$m.

**Revendications pour les Etats contractants suivants : ES, GR**

1. Procédé de fabrication d'une composition thérapeutique, utile notamment dans le traitement par voie orale des hyperlipidémies et des hypercholestérolémies et renfermant du fénofibrate et un agent tensio-actif, ledit procédé étant caractérisé en ce qu'il comprend la co-micronisation du fénofibrate et de l'agent tensio-actif solide.

2. Procédé selon la revendication 1, caractérisé en ce que le rapport pondéral agent tensio-actif/fénofibrate est compris entre 0,75/100 et 10,5/100.

3. Procédé selon la revendication 1, caractérisé en ce que la quantité de fénofibrate est égale à 200 mg par unité thérapeutique.

4. Procédé selon la revendication 1, caractérisé en ce que l'agent tensio-actif solide est le laurylsulfate de sodium.

5. Procédé selon la revendication 4, caractérisé en ce que la quantité de laurylsulfate de sodium est comprise entre 0,5 et 7 % en poids par rapport au poids total de la formulation.

6. Procédé selon la revendication 1, dans lequel la taille moyenne des particules de fénofibrate et de laurylsulfate de sodium co-micronisés est inférieure à 15 $\mu$m, de préférence inférieure ou égale à 10 $\mu$m, et mieux inférieure ou égale à 5 $\mu$m.

7. Procédé selon la revendication 1 caractérisé en ce que :
(i) on mélange intimement puis co-micronise le fénofibrate et un agent tensio-actif solide,
(ii) on additionne au mélange obtenu du lactose et de l'amidon,
(iii) on granule l'ensemble en présence d'eau,
(iv) on sèche jusqu'à obtention d'un granulé contenant au plus 1 % d'eau,
(v) on calibre le granulé,

7

(vi) on additionne de la polyvinylpyrrolidone et du stéarate de magnésium,

8. Procédé pour améliorer la biodisponibilité du fénofibrate in vivo, ledit procédé étant caractérisé en ce qu'il comprend la co-micronisation du fénofibrate et d'un agent tensio-actif solide, ladite co-micronisation étant réalisée par micronisation d'un mélange fénofibrate/agent tensio-actif solide jusqu'à obtention d'une poudre de granulométrie à 15 µm et mieux inférieure ou égale à 5 µm.

**Patentansprüche**

**Patentansprüche für folgende Vertragsstaaten : AT, BE, CH, DE, FR, GB, IT, LI, LU, NL, SE**

1. Therapeutische Zusammensetzung, insbesondere für die orale Behandlung von Hyperlipidämien und Hypercholesterinämien , dadurch gekennzeichnet, daß sie Fenofibrat und ein festes, oberflächenaktives Mittel, die einer gemeinsamen Strahlmühlenbehandlung unterzogen worden sind, enthält.

2. Therapeutische Zusammensetzung gemäß Anspruch 1, dadurch gekennzeichnet, daß das Gewichtsverhältnis oberflächenaktives Mittel/Fenofibrat zwischen 0,75/100 und 10,5/100 liegt.

3. Therapeutische Zusammensetzung gemäß Anspruch 1, dadurch gekennzeichnet, daß die Menge an Fenofibrat 200 mg je therapeutische Einheit beträgt.

4. Therapeutische Zusammensetzung gemäß Anspruch 1, dadurch gekennzeichnet, daß das feste, oberflächenaktive Mittel Natriumlaurylsulfat ist.

5. Therapeutische Zusammensetzung gemäß Anspruch 4, dadurch gekennzeichnet, daß die Menge an Natriumlaurylsulfat zwischen 0,5 und 7 Gew.%, bezogen auf das Gesamtgewicht der Formulierung, beträgt.

6. Therapeutische Zusammensetzung gemäß Anspruch 1, dadurch gekennzeichnet, daß die durchschnittliche Größe der einer gemeinsamen Strahlmühlenbehandlung unterzogenen Teilchen von Fenofibrat und Natriumlaurylsulfat geringer als 15 µm, vorzugsweise geringer oder gleich 10 µm und insbesondere geringer oder gleich 5 µm, ist.

7. Therapeutische Zusammensetzung gemäß einem der Ansprüche 1 bis 6, dadurch gekennzeichnet, daß sie außerdem Exzipienten, wie Dispersionsmitteln, Füllstoffe und Fließmittel enthält.

8. Verfahren zur Herstellung einer therapeutischen Zusammensetzung gemäß Anspruch 1, dadurch gekennzeichnet , daß man
   (i) das Fenofibrat und ein festes, oberflächenaktives Mittel innig mischt und hiernach einer gemeinsamen Strahlmülenbehandlung unterzieht,
   (ii) zu dem erhaltenen Gemisch Lactose und Stärke zugibt,
   (iii) das Ganze in Gegenwart von Wasser granuliert,
   (iv) bis zur Erzielung eines Granulats, das höchstens 1% Wasser enthält, trocknet,
   (v) das Granulat kalibriert, und
   (vi) Polyvinylpyrrolidon und Magnesiumstearat zusetzt.

9. Verfahren gemäß Anspruch 8, dadurch gekennzeichnet, daß außerdem die durchschnittliche Größe der einer gemeinsamen Strahlmühlenbehandlung unterzogenen Teilchen von Fenofibrat und Natriumlaurylsulfat geringer als 15 µm ist.

10. Verfahren zur Verbesserung der Bioverfügbarkeit von Fenofibrat in vivo, dadurch gekennzeichnet, daß es die gemeinsame Strahlmühlenbehandlung von Fenofibrat und einem festen, oberflächenaktiven Mittel umfaßt, wobei die gemeinsame Strahlmühlenbehandlung durch Strahlmühlenbehandlung eines Fenofibrat/festes, oberflächenaktives Mittel-Gemisches bis zur Erzielung eines Pulvers mit einer Teilchengröße von geringer als 15 µm und vorzugsweise geringer oder gleich 5 µm durchgeführt wird.

**Patentansprüche für folgende Vertragsstaaten : ES, GR**

1. Verfahren zur Herstellung einer therapeutischen Zusammensetzung, insbesondere verwendbar bei der oralen Behandlung von Hyperlipidämien und Hypercholesterinämien, enthaltend Fenofibrat und ein oberflächenaktives Mittel, dadurch gekennzeichnet, daß es die gemeinsame Strahlmühlenbehandlung von Fenofibrat und dem festen, oberflächenaktiven Mittel umfaßt.

2. Verfahren gemäß Anspruch 1, dadurch gekennzeichnet , daß das Gewichtsverhältnis oberflächenaktives Mittel/Fenofibrat zwischen 0,75/100 und 10,5/100 liegt.

3. Verfahren gemäß Anspruch 1, dadurch gekennzeichnet, daß die Menge des Fenofibrats 200 mg je therapeutische Einheit entspricht.

4. Verfahren gemäß Anspruch 1, dadurch gekennzeichnet, daß das feste, oberflächenaktive Mittel Natriumlaurylsulfat ist.

5. Verfahren gemäß Anspruch 4, dadurch gekennzeichnet , daß die Menge an Natriumlaurylsulfat zwischen 0,5 und 7 Gew.%, bezogen auf das Gesamtgewicht der Formulierung, beträgt.

6. Verfahren gemäß Anspruch 1, worin die durchschnittliche Größe der gemeinsam einer Strahlmühlenbehandlung unterzogenen Teilchen von Fenofibrat und Natriumlaurylsulfat geringer als 15 $\mu$m, vorzugsweise geringer oder gleich 10 $\mu$m und insbesondere geringer oder gleich 5 $\mu$m, ist.

7. Verfahren gemäß Anspruch 1, dadurch gekennzeichnet , daß man
(i) das Fenofibrat und ein festes, oberflächenaktives Mittel innig mischt und hiernach einer gemeinsamen Strahlenbehandlung unterzieht,
(ii) zu dem erhaltenen Gemisch Lactose und Stärke zugibt,
(iii) das Ganze in Anwesenheit von Wasser granuliert,
(iv) bis zur Erzielung eines höchstens 1% enthaltenden Granulats trocknet,
(v) das Granulat kalibriert,
(vi) man Polyvinylpyrrolidon und Magnesiumstearat zusetzt.

8. Verfahren zur Verbesserung der Bioverfügbarkeit von Fenofibrat in vivo, dadurch gekennzeichnet, daß es die gemeinsame Strahlmühlenbehandlung von Fenofibrat und einem festen, oberflächenaktiven Mittel umfaßt, wobei die gemeinsame Strahlmühlenbehandlung durch Strahlmühlenbehandlung eines Fenofibrat/festes, oberflächenaktives Mittel-Gemisches bis zur Erzielung eines Pulvers mit einer Teilchengröße von 15 $\mu$m und vorzugsweise geringer oder gleich 5 $\mu$m durchgeführt wird.

**Claims**

**Claims for the following Contracting States : AT, BE, CH, DE, FR, GB, IT, LI, LU, NL, SE**

1. A therapeutic composition, which is useful especially in the oral treatment of hyperlipidemia and hypercholesterolemia, said composition containing fenofibrate and a solid surfactant which have been co-micronized.

2. The therapeutic composition according to claim 1, wherein the weight ratio surfactant/fenofibrate is comprised between from 0.75/100 to 10.5/100.

3. The therapeutic composition according to claim 1, wherein the amount of fenofibrate is equal to 200 mg per therapeutic unit.

4. The therapeutic composition according to claim 1, wherein the solid surfactant is sodium laurylsulfate.

5. The therapeutic composition according to claim 4, wherein the amount of sodium laurylsulfate is comprised between from 0.5 to 7 % by weight, relative to the total weight of the formulation.

6. The therapeutic composition according to claim 1, wherein the mean particle size of the co-micronized fenofibrate and sodium laurylsulfate is less than 15 $\mu$m, preferably less than or equal to 10 $\mu$m and par-

ticularly preferably less than or equal to 5 μm.

7. The therapeutic composition according to any one of claims 1 to 6, which also contains excipients such as dispersants, fillers and flow enhancers.

8. A method for the manufacture of a therapeutic composition according to claim 1, which comprises :
    (i) intimately mixing and then co-micronizing the fenofibrate and a solid surfactant,
    (ii) adding lactose and starch to the mixture obtained,
    (iii) converting the whole to granules in the presence of water,
    (iv) drying the granules until they contain no more than 1 % of water,
    (v) grading the granules, and
    (vi) adding polyvinylpyrrolidone and magnesium stearate.

9. The method according to claim 8, wherein the mean particle size of the co-micronized fenofibrate and sodium laurylsulfate is less than 15 μm.

10. A method for improving the bioavailability of fenofibrate in vivo, which comprises co-micronization of the fenofibrate and a solid surfactant, the said co-micronization being carried out by micronization of a fenofibrate/solid surfactant mixture until the particle size of the powder obtained is less than 15 μm and preferably less than or equal to 5 μm.

**Claims for the following Contracting States : ES, GR**

1. A method for the preparation of therapeutic composition which is useful especially in the oral treatment of hyperlipidemia and hypercholesterolemia and contains fenofibrate and a surfactant, said method comprising co-micronizing of said fenofibrate and a solid surfactant which have been co-micronized.

2. A method according to claim 1, wherein the weight ratio surfactant/fenofibrate is comprised between from 0.75/100 to 10.5/100.

3. A method according to claim 1, wherein the amount of fenofibrate is equal to 200 mg per therapeutic unit.

4. A method according to claim 1, wherein the solid surfactant is sodium laurylsulfate.

5. A method according to claim 4, wherein the amount of sodium laurylsulfate is comprised between from 0.5 to 7 % by weight, relative to the total weight of the formulation.

6. A method according to claim 1, wherein the mean particle size of the co-micronized fenofibrate and sodium laurylsulfate is less than 15 μm, preferably less than or equal to 10 μm and particularly preferably less than or equal to 5 μm.

7. A method according to claim 1, which comprises :
    (i) intimately mixing and then co-micronizing the fenofibrate and a solid surfactant,
    (ii) adding lactose and starch to the mixture obtained,
    (iii) converting the whole to granules in the presence of water,
    (iv) drying the granules until they contain no more than 1 % of water,
    (v) grading the granules, and
    (vi) adding polyvinylpyrrolidone and magnesium stearate.

8. A method for improving the bioavailability of fenofibrate in vivo, which comprises co-micronization of said fenofibrate and a solid surfactant, said co-micronization being carried out by micronization of a fenofibrate/solid surfactant mixture until the particle size of the powder obtained is less than 15 μm and preferably less than or equal to 5 μm.